# FASCICULE DE BREVET EUROPEEN

(11) **EP 1 215 960 B1**
(45) Date de publication et mention de la délivrance du brevet: **01.03.2006**
(21) Numéro de dépôt: 00966229.7
(22) Date de dépôt: 29.09.2000
(51) Int. Cl.: A01K 67/027

(54) **METHODE DE RECONSTITUTION D'UN EMBRYON DE MAMMIFERE NON HUMAIN AVEC DES CELLULES DIFFERENCIEES**
VERFAHREN ZUR WIEDERHERSTELLUNG EINES NICHT-MENSCHLISCHEN EMBRYOS MIT DIFFERENZIERTEN ZELLEN
METHOD FOR RECONSTRUCTING A NON-HUMAN MAMMAL EMBRYO WITH FOETAL ADULT DIFFERENTIATED CELLS

(30) Priorité: 01.10.1999 FR 9912287
(43) Date de publication de la demande: 26.06.2002
(73) Titulaire: INSTITUT NATIONAL DE LA RECHERCHE AGRONOMIQUE, 75007 Paris (FR)
(72) Inventeur: RENARD, Jean-Paul, F-92170 Vanves (FR); VIGNON, Xavier, F-78000 Versailles (FR)
(74) Mandataire: Goulard, Sophie
(86) Numéro de dépôt international: PCT/FR2000/002698
(87) Numéro de publication internationale: WO 2001/024624

(56) Documents cités:
- VIGNON X ET AL.: "Development potential of bovine embryos reconstructed from enucleated matured oocytes fused with cultured somatic cells" COMPTES RENDUS DE L ACADEMIE DES SCIENCES, vol. 321, 1998, pages 735-745, XP000914895 SCIENTIFIQUES & MEDICALES ELSEVIER FR cité dans la demande
- RENARD JP, ET AL.: "Lymphoid hypoplasia and somatic cloning." LANCET., vol. 353, no. 9163, 1 mai 1999 (1999-05-01), pages 1489-1491, XP000914897 cité dans la demande
- VIGNON X ET AL.: " Development of bovine nuclear transfer embryos reconstituted with quiescent and proliferative skin fibroblasts" THERIOGENOLOGY, vol. 51, 1 janvier 1999 (1999-01-01), page 216 XP000921428 & International Workshop on Embryogenesis and Implantation; Kamuela, Hawaii, USA; February 2-4, 1999
- CAMPBELL K H S ET AL: "NUCLEAR-CYTOPLASMIC INTERACTIONS DURING THE FIRST CELL CYCLE OF NUCLEAR TRANSFER RECONSTRUCTED BOVINE ENBRYOS: IMPLICATIONS FOR DEOXYRIBONUCLEIC ACID REPLICATION AND DEVELOPMENT" BIOLOGY OF REPRODUCTION,US,SOCIETY FOR THE STUDY OF REPRODUCTION, CHAMPAIGN, IL, vol. 49, no. 5, 1 novembre 1993 (1993-11-01), pages 933-942, XP000604579 ISSN: 0006-3363 cité dans la demande
- ADENOT PG, MERCIER Y, RENARD JP, THOMPSON EM.: "Differential H4 acetylation of paternal and maternal chromatin precedes DNA replication and differential transcriptional activity in pronuclei of 1-cell mouse embryos." DEVELOPMENT., vol. 124, no. 22, novembre 1997 (1997-11), pages 4615-4625, XP000922597 cité dans la demande
- FANG ZHEN SUN & ROBERT M. MOOR: "Nuclear transplantation in mammalian eggs and embryos" CURRENT TOPICS IN DEVELOPMENTAL BIOLOGY, vol. 30, 1995, pages 147-176, XP000921085
- BROWN RL, CLARK RW, CHIU JF, STUBBLEFIELD E.: "Protease activation of G1 nuclei isolated from Chinese hamster fibroblasts." EXP CELL RES., vol. 104, no. 1, janvier 1977 (1977-01), pages 207-213, XP000922636 cité dans la demande
- KRAEMER RJ, COFFEY DS.: "The interaction of natural and synthetic polyanions with mammalian nucleo. II. Nuclear swelling." BIOCHIM BIOPHYS ACTA. 1970, vol. 224, no. 2, 14 décembre 1970 (1970-12-14), pages 568-578, XP000922644 cité dans la demande
- LIU L, JU JC, YANG X.: "Parthenogenetic development and protein patterns of newly matured bovine oocytes after chemical activation." BIOL REPROD. 1998 APR;58(4):952-62., vol. 584, avril 1998 (1998-04), pages 952-962, XP000922640 cité dans la demande

## Description

La présente invention concerne la reconstitution d'embryons de mammifères non-humains par transfert nucléaire.

Les techniques de production d'animaux non-humains, notamment de mammifères, à partir d'embryons reconstitués par transfert du noyau d'une cellule donneuse somatique dans le cytoplasme d'un ovocyte receveur préalablement énucléé font actuellement l'objet d'un grand intérêt, du fait de leurs applications potentielles. Parmi celles ci, on peut citer notamment :
- la transgénèse animale: l'intégration d'un gène d'intérêt dans des cellules en culture, suivie du transfert des noyaux de celles-ci dans des ovocytes receveurs pourrait permettre d'augmenter l'efficacité de la transgenèse ;
- la possibilité de multiplier des animaux possédant des caractéristiques génétiques particulières, que celles-ci soient naturelles ou résultent de la transgénèse ;
- l'évaluation génétique : le fait de disposer de plusieurs animaux dotés d'un patrimoine génétique identique peut permettre d'évaluer l'influence respective des facteurs génétiques et environnementaux sur les qualités d'un animal;
- la possibilité d'obtenir des lignées de cellules embryonnaires pouvant ensuite être différenciées in vitro.

Les principaux obstacles rencontrés pour la mise au point de techniques de reconstitution d'embryon par transfert nucléaire proviennent de la difficulté de coordonner les interactions noyau donneur/cytoplasme receveur, dont dépend le développement futur de l'embryon. Les méthodes de transfert nucléaire comprennent donc classiquement, préalablement au transfert du noyau, la préparation de la cellule donneuse et/ou de l'ovocyte receveur.

Les ovocytes receveurs généralement utilisés résultent de l'énucléation d'ovocytes au stade métaphase II, après maturation in vitro. A ce stade du cycle cellulaire le cytoplasme possède une activité MPF (Maturation Promoting Factor) importante, qui décroît progressivement à partir du moment où le cytoplasme est activé. Cette activation, qui résulte de l'entrée d'un spermatozoïde dans le cas de la fécondation naturelle, peut être induite artificiellement dans le cas du transfert de noyau.

Lorsque le transfert du noyau et l'activation du cytoplasme receveur interviennent simultanément (ce qui se produit par exemple lorsque la fusion du noyau et du cytoplasme est effectuée par une impulsion électrique suffisante pour stimuler ce dernier), l'activité MPF élevée induit la dégradation de l'enveloppe nucléaire, la condensation de la chromatine exposant, immédiatement après la fusion, le matériel nucléaire à un environnement cytoplasmique métaphasique. Il a été rapporté [CAMPBELL et al., Biol. Reprod., 49, 933-942, (1993)] que ces phénomènes risquent d'entraîner des anomalies chromosomiques, notamment dans le cas où le noyau donneur est en phase G2 ou S du cycle cellulaire.

Afin d'éviter la condensation prématurée de la chromatine et de conserver l'enveloppe nucléaire intacte, il a été proposé [BARNES et al., Mol. Reprod. Dev., 36, 33-41, (1993) ; STICE et al., Mol. Reprod. Dev., 38, 61-68, (1994)], d'activer préalablement le cytoplasme receveur, et de n'effectuer le transfert du noyau que lorsqu'un environnement cytoplasmique de type interphasique a été obtenu, ce qui se traduit notamment par un faible niveau d'activité MPF, ou bien d'utiliser un cytoplasme receveur provenant d'ovocytes âgés [CHESNE et al., CRAS, 316, 487-492, (1993)], plus sensibles à l'activation et dans lesquels la transition vers l'interphase se produit plus rapidement.

Cette approche permet d'améliorer les taux de succès lorsque l'on utilise des cellules embryonnaires (blastomères) comme cellules donneuses. En revanche, notamment chez les bovins, elle est beaucoup moins efficace dans le cas de noyaux provenant de noyaux de cellules somatiques différenciées où les taux de développement des embryons demeurent très faibles [VIGNON et al., C. R. Acad. Sci. Paris, 321, 735-745, (1998) ; RENARD et al., The Lancet, 353, 1489-1491, (1999)].

Selon une autre approche, l'exposition du noyau donneur issu d'une cellule différenciée aux facteurs du cytoplasme receveur en métaphase aurait pour effet de permettre la reprogrammation du noyau, et de restaurer sa totipotence. La Demande Internationale PCT WO 97/07668, au nom du ROSLIN INSTITUTE (Inventeurs: WILMUT et al.) propose ainsi de n'activer les ovocytes que plusieurs heures après la fusion, afin de prolonger le contact entre le matériel nucléaire issu de la cellule donneuse et le cytoplasme receveur. Pour maintenir une ploïdie correcte dans ces conditions, il est nécessaire d'utiliser des cellules donneuses en phase G0 ou G1, et de stabiliser ou d'inhiber la formation des microtubules pendant l'activation.

La Demande Internationale PCT WO 97/07669, au nom du ROSLIN INSTITUTE (Inventeurs : WILMUT et al.) préconise l'utilisation de noyaux obtenus à partir de cellules préalablement amenées à l'état de quiescence (phase G₀ du cycle cellulaire) par une période de culture en absence de sérum ; ce traitement faciliterait également la reprogrammation du noyau donneur, en le rendant plus réceptif à des facteurs cytoplasmiques de l'ovocyte receveur. Cette approche a permis, notamment chez les ovins, d'améliorer le taux de développement d'embryons obtenus à partir de noyaux de cellules différenciées.

Chez la souris, WAKAMAYA et al. (Nature, 394, 369-374, 1998) rapportent le développement d'embryons obtenus à partir de noyaux en phase G₀ ou G₁ de cellules de cumulus différenciées injectés dans le cytoplasme d'ovocytes en métaphase II activés 1 à 3 heures après l'injection.

Les Inventeurs ont maintenant mis au point une nouvelle méthode de reconstitution *in vitro* d'embryons de mammifères non-humains, permettant d'améliorer les taux d'obtention et de développement d'embryons viables à partir de noyaux de cellules somatiques issus de différents tissus foetaux ou adultes.

Dans ce cadre, la présente invention a pour objet une méthode de traitement du noyau diploïde d'une cellule somatique préalablement à son transfert dans le cytoplasme d'un ovocyte receveur, ledit traitement comprenant :
a) la protéolyse ménagée des protéines non-histones, et
b) l'induction d'un gonflement isomorphe dudit noyau.

Ce traitement a pour effet de rendre l'ADN nucléaire plus accessible et plus réactif à l'environnement cytoplasmique où se trouvent des facteurs capables d'interagir avec les structures du noyau même lorsqu'il n'y a pas de rupture de l'enveloppe nucléaire [ADENOT et al., Development, 124, 4615-4625, (1997) ; THOMPSON et al., Dev. Genetics, 42, 22-31, (1998)]. La préservation de la membrane du noyau permet le maintien d'une ploïdie correcte avant la première division de l'embryon.

Le noyau diploïde peut notamment être obtenu à partir de cultures primaires de cellules foetales ou de cellules adultes originaire de différents tissus (par exemple des épithéliums de glande mammaire ou de peau, des cellules musculaires, des cellules hépatiques, etc.). Les cellules peuvent être indifféremment issues d'une culture primaire fraîche, ou bien d'une culture établie sur plusieurs passages ou redémarrée à partir de cellules conservées par congélation. Les cellules peuvent être utilisées quelle que soit la phase du cycle cellulaire dans laquelle elles se trouvent.

Les noyaux peuvent être prélevés à partir de ces cellules donneuses, traités conformément à l'invention, et transférés ensuite par micro-injection dans le cytoplasme de l'ovocyte receveur ; il est cependant plus commode, en pratique, de traiter les noyaux contenus dans les cellules donneuses et d'effectuer ensuite le transfert nucléaire par fusion de la cellule donneuse et du cytoplasme receveur.

Dans ce dernier cas, la mise en oeuvre de la méthode de l'invention nécessite une étape préalable de perméabilisation de la membrane cytoplasmique de la cellule donneuse, afin de rendre le noyau accessible à l'action des traitements.

La perméabilisation des membranes plasmiques peut par exemple être obtenue par incubation de la cellule avec un ou plusieurs agents perméabilisants doux, tels que la lysolécithine, la streptolysine, la saponine, la digitonine, à condition que les conditions d'utilisation n'induisent pas la lyse des cellules et préservent à la membrane plasmique un état compatible avec l'opération ultérieure de fusion avec l'ovocyte receveur. Les doses utilisables et les conditions d'incubation varient d'une source de cellules à l'autre. On détermine les conditions adéquates en testant préalablement la perméabilisation à l'aide de bleu Trypan ; les cellules perméabilisées prennent une coloration bleue facilement observable au microscope. Les conditions adéquates correspondent à celles qui donnent entre 50 et 100% de cellules perméables. Le traitement de protéolyse et préalablement à l'induction du gonflement des noyaux, qui demande une incubation plus longue.

Pour la protéolyse ménagée, on utilisera une protéase à sérine, telle que la trypsine ou la chymotrypsine ; l'action de la protéase doit se limiter à la dégradation de protéines non-histones du noyau, et de protéines du cytosquelette entourant le noyau, et ne doit pas conduire à la lyse des noyaux. Généralement on utilisera des concentrations de protéase très faibles. Par exemple, dans le cas d'une protéolyse effectuée sur des cellules entières (en même temps que la perméabilisation ou consécutivement à celle-ci), on peut utiliser des concentrations de trypsine de l'ordre de 1 à 10 U/ml dans le mélange d'incubation des cellules, pour une durée d'incubation variant de 1 à 10 min. à 37°C. Dans le cas du traitement de noyaux isolés, les doses de protéase et les temps d'incubation devront être encore réduits.

En outre, la trypsine possède un effet direct d'activation sur des ADN polymérases dans les cellules qui sont en phase G1 ou G0 lors du traitement [BROWN et al., Exp. Cell Res., 104, 207-213, (I977)]. Ceci permettrait de faciliter la reprise du cycle cellulaire des noyaux qui se trouvent en phase G0/G1 lors de leur incorporation dans un cytoplasme receveur en interphase.

Le gonflement du noyau peut être induit par un traitement à l'aide d'au moins un composé polyanionique, tel que l'héparine, le dextrane sulfate, ou les acides polyaspartiques de poids moléculaire élevé (>20 000), comme décrit par KRAEMER et COFFEY [Biochim. Biophys. Acta., 224, 568-578, (1970)].

Le traitement est effectué, après la protéolyse ou simultanément à celle-ci, par incubation des noyaux ou des cellules les contenant en présence du polyanion, jusqu'à ce qu'un gonflement du noyau soit observé. Par exemple, dans le cas des cellules entières observé. Par exemple, dans le cas des cellules entières perméabilisées on peut utiliser entre 50 et 200 µg de polyanion par ml de milieu d'incubation, pour une incubation de 30 à 60 min à température ambiante (15 à 25°C) .

A l'issue de ce traitement, on effectue le transfert du noyau dans le cytoplasme de l'ovocyte receveur.

Le transfert des noyaux traités conformément à l'invention peut être effectué quel que soit le stade du cycle cellulaire des cellules donneuses, et lorsque le cytoplasme de l'ovocyte receveur est en métaphase II ou en interphase. Dans le cas de fusion des noyaux avec des ovocytes receveurs en métaphase II (20 à 25 h après le début de la maturation), il faudra réaliser une activation des embryons reconstitués, par exemple, par un procédé d'activation chimique tel qu'il a été décrit par LIU et al. [Mol. Reprod. Dev., 49, 298-307, (1998)], ou par toute autre méthode.

Il est toutefois particulièrement intéressant d'utiliser des cytoplasmes d'ovocytes receveurs préalablement préparés de façon à les amener en interphase.

On définit comme « cytoplasme receveur en interphase » un cytoplasme dans lequel le taux de MPF est inférieur à celui auquel il induit la dégradation de la membrane nucléaire et la condensation de la chromatine.

Ce cytoplasme peut être obtenu à partir d'un ovocyte maturé in vivo ou in vitro. Le matériel chromosomique est retiré par micromanipulation alors que l'ovocyte est bloqué au stade métaphase II. Le cytoplasme résultant est ensuite traité pour réduire l'activité MPF. Il peut par exemple être préparé par une méthode de vieillissement *in vitro* et de refroidissement avant la fusion [CHESNE et al., C. R. Acad. Sci., 316, 487-491, (1993)]. Il est également possible d'obtenir le même résultat à l'aide de drogues inhibant les synthèses protéiques (cycloheximide) ou de drogues inhibant les phosphorylations (6-DMAP) ou d'un mélange des 2 drogues. Dans ce cas, l'état d'interphase est généralement obtenu en 1 à 4 h d'incubation.

La reconstitution de l'embryon peut être effectuée, dans le cas de noyaux isolés, par micro-injection. Il est toutefois particulièrement avantageux d'utiliser directement des cellules traitées pour les fusionner avec les cytoplasmes d'ovocytes receveurs. Dans ce cas la fusion est de préférence, réalisée par choc électrique, ou par toute autre méthode. Il n'est pas nécessaire d'opérer dans des conditions destinées à induire l'activation du cytoplasme receveur.

Les embryons reconstitués sont directement cultivés in vitro sans autre activation après la fusion, jusqu'à leur développement au stade blastocyste.

Les noyaux donneurs ne subissent pas de destruction de leur membrane nucléaire, ni de condensation de la chromatine (PCC) avant la première division qui intervient après la fusion.

Lorsque les embryons ont atteint le stade blastocyte, ils sont ensuite transférés dans des femelles receveuses, selon les techniques classiques connues en elles-mêmes.

La méthode conforme à l'invention permet d'une part d'améliorer la reprogrammation des noyaux transférés en permettant l'accès des facteurs cytoplasmiques à la chromatine grâce à une action enzymatique modérée avant la fusion, et d'autre part de favoriser le maintien d'une ploïdie correcte des cellules embryonnaires en minimisant la condensation de la chromatine et en empêchant sa dispersion lors du remodelage du noyau introduit dans le cytoplasme receveur. Cette modification dans les étapes initiales des relations entre le noyau et le cytoplasme permet de favoriser le développement à terme des embryons reconstitués à partir de noyaux de cellules somatiques différenciées, et ce quelle que soit la phase du cycle cellulaire dans laquelle se trouvent ces cellules.

Elle peut s'appliquer à différentes espèces de mammifères non-humains ; elle est toutefois plus particulièrement adaptée à la reconstitution d'embryons de mammifères ongulés, notamment de ruminants, et en particulier d'ovins, de bovins, de caprins ou de porcins.

La présente invention sera mieux comprise à l'aide du complément de description qui va suivre, qui se réfère à des exemples non-limitatifs de mise en oeuvre du procédé conforme à l'invention pour la reconstitution d'embryons bovins.

### EXEMPLE 1:

### Préparation des cellules et reconstitution des embryons.

Les cellules donneuses sont obtenues à partir de cultures de cellules foetales de peau ou de muscle, ou de cultures de biopsie d'oreille d'animaux adultes (cellules de peau) préparées comme décrit par VIGNON et al., (C. R. Acad. Sci. Paris, 321, 735-745, 1998). Dans ces conditions, on obtient des cellules différenciées, de type fibroblaste.

Les boîtes de cellules sont vidées du milieu de culture, rincées par du tampon PBS et remplies d'un milieu dit de perméabilisation dont la composition est la suivante: solution saline équilibrée de HANKS sans calcium et sans magnésium, contenant 0,5 µg/ml de trypsine, et 20 à 30 µg/ml de lysolécithine dans le cas des cellules foetales de peau et 15 à 20 µg/ml de lysolécithine dans le cas des cellules de peau d'animaux adultes. Après une incubation de 5 min à 37°C, les boites sont vidées du milieu d'incubation. Pour stopper la perméabilisation et la protéolyse, les boites sont immédiatement rincées avec une solution tampon (solution saline équilibrée de HANKS sans calcium et sans de HANKS sans calcium ni magnésium contenant 10 U/ml d'héparine. L'incubation est réalisée pendant 30 à 60 min à température ambiante. Les cellules sont ensuite collectées par grattage du support de culture, centrifugées à 1200 g pendant 5 min, et resuspendues dans du milieu de culture dépourvu de sérum préalablement à la fusion avec les ovocytes receveurs.

Les ovocytes receveurs sont énucléés, et amenés en interphase selon le protocole décrit par VIGNON et al. [C. R. Acad. Sci. Paris, 321, 735-745, (1998)].

Le transfert de noyau est effectué en introduisant une cellule donneuse isolée sous la zone pellucide du cytoplasme receveur, et en effectuant la fusion dans les conditions suivantes : 2 pulses électriques de 2,2 kV/cm d'une durée de 20 µs dans du milieu TCM199 (LIFE TECHNOLOGIES, Cergy Pontoise, France) additionné de 5 µg/ml de cytochalasine B.

La fusion est vérifiée par observation au microscope binoculaire, et les embryons reconstitués sont mis en culture *in vitro,* puis implantés *in vivo,* comme décrit par VIGNON et al. [C. R. Acad. Sci. Paris, 321, 735-745, (1998)].

### Témoins :

Des témoins sont obtenus selon le protocole décrit ci-dessus, mais sans perméabilisation, ni traitement à la trypsine et à l'héparine des cellules donneuses.

### Résultats :

Dans le cas des cellules foetales, de peau ou de muscle, 47 blastocystes ont été obtenus après avoir reconstitué 663 embryons (7,1%) avec des cellules donneuses ayant subi le traitement conforme à l'invention. En comparaison, avec les cellules témoin, 13 blastocystes ont été obtenus pour 386 embryons reconstitués (3,37%).

Après transplantation des embryons obtenus à partir des cellules donneuses ayant subi le traitement conforme à l' invention, 27 vaches receveuses ont donné 5 gestations de plus de 90 jours et 4 animaux viables ont vu le jour. Pour les cellules témoin, 6 vaches receveuses ont été transplantées et seule une gestation est allée au delà de 90 jours, mais sans atteindre le terme (avortement tardif à 8 mois de gestation).

Dans le cas des cellules issues de biopsies d'oreille d'animaux adultes, 23 blastocystes ont été produits après 580 reconstructions d'embryons (4%) avec des cellules donneuses ayant subi le traitement conforme à l'invention, alors que 6 blastocystes ont été obtenus sur 250 reconstructions (2,4%) avec des cellules témoin. Tous les embryons ont été transplantés, et une naissance a été obtenue, à partir de la série des embryons issus de cellules traitées.

### EXEMPLE 2 :

Lors d'une autre série d'expérimentations, des embryons ont été reconstitués, selon le protocole décrit dans l'exemple 1 ci-dessus, à partir de cellules somatiques bovines foetales ou adultes issues de cultures en prolifération, ou bien issues de cultures mises en état de quiescence (par maintien, pendant les 36 à 48 heures précédant leur utilisation, dans un milieu dépourvu de sérum). Des embryons témoin ont également été reconstitués comme décrit à l'exemple 1 ci-dessus.

Les résultats sont illustrés par les tableaux I et II ci-après. Le tableau I représente les résultats obtenus avec les cellules donneuses issues de cultures en prolifération.

**TABLEAU 1**

| Cellules donneuses | Nombre d'embryons | | Nombre de Morula (%) | Nombre de Blastocystes (%) |
|---|---|---|---|---|
| | Reconstitués (%) | Clivés (%) | | |
| Traitées | 502 (56,5) | 256 (51,0) | 43 (8,6) | 28 (5,6) |
| Témoins | 221 (55,6) | 115 (52,0) | 10 (4,5) | 6 (2,7) |

Le tableau II représente les résultats obtenus avec les cellules donneuses quiescentes.

**TABLEAU II**

| Cellules donneuses | Nombre d'embryons | | Nombre de Morula (%) | Nombre de Blastocystes (%) |
|---|---|---|---|---|
| | Reconstitués (%) | Clivés (%) | | |
| Traitées | 717 (58,9) | 337 (47,0) | 83 (11,6) | 48 (6,7) |
| Témoins | 280 (69,3) | 168 (60,0) | 13 (4,6) | 7 (2,5) |

Ces résultats montrent que dans le cadre de l'utilisation d'un cytoplasme receveur en interphase, l'état initial des cellules donneuses (quiescence ou prolifération) n'a pas d'influence significative sur le taux de développement des embryons ; en revanche, le traitement des cellules donneuses conformément à l'invention augmente significativement ce taux de développement.

Les blastocystes obtenus à l'issue des 2 expérimentations ci dessus ont été transférés chez des vaches receveuses. Les résultats sont illustrés par le tableau III ci-dessous.

**TABLEAU III**

| Cellules | Nombre de Blastocystes transférés | Nombre de gestations/Nombre de receveuses | | | Naissances |
|---|---|---|---|---|---|
| | | D 35 | D 60 | D>90 | |
| Traitées | 76 | 7/50 (14,0) | 6/50 (12,0) | 6/50 (12,0) | 5 (10,0) |
| Non-traitées | 13 | 3/8 (37,5) | 1/8 (12,5) | 1/8 (12,5) | 0 (0,0) |

Ces résultats confirment que le traitement des cellules donneuses conformément à l'invention augmente significativement le taux d'obtention d'embryons pouvant donner naissance à des animaux viables.

### EXEMPLE 3 :

Les cellules donneuses sont des fibroblastes en prolifération issus de culture de cellules de peau de foetus bovins ou adultes. Elles sont préparées comme dans l'exemple 1. La perméabilisation est réalisée par incubation en présence de 15 à 20 µg/ml de lysolécithine.

Les ovocytes receveurs sont énuclées après 22 à 24h de maturation *in vitro,* et fusionnés à 24-25h avec les cellules donneuses dans les mêmes conditions que celles décrites dans l'exemple 1. La fusion est suivie d'une activation chimique selon un protocole décrit par LIU et al. [Mol. Reprod. Dev., 49, 298-307, (1998)] : immédiatement après fusion, les embryons reconstitués sont incubés en présence de 10 µg/ml de cycloheximide et 5 µg/ml de cytochalasine B dans du milieu TCM 199 (LIFE TECHNOLOGIES, Cergy Pontoise, France) pendant 5h. Les embryons sont ensuite mis en culture *in vitro*. Des embryons témoins ont été reconstitués de la même façon, mais sans traitement des cellules donneuses préalablement à la fusion.

Les résultats sont illustrés par les tableaux IV et V ci-dessous :

**TABLEAU IV**

| Cellules donneuses | Embryons reconstitués (%) | Embryons clivés (%) | Morula (%) | Blastocystes (%) |
|---|---|---|---|---|
| Traitées | 365 (53,7) | 227 (62,2) | 101 (27,7) | 88 (24,1) |
| Témoins | 438 (59,2) | 271 (61,9) | 101 (21,1) | 70 (16,0) |

**TABLEAU V**

| Cellules donneuses | Blastocystes transférés | Gestation/rec D35 | Gestation/rec D60 | Gestation/rec D90 | Naissances |
|---|---|---|---|---|---|
| Traitées 60 | 60 | 18/40 (45,0) | 17/40 (42,5) | 9/40 (22,5) | 4/40 (10,0) |
| Témoins 55 | 55 | 10/38 (26,3) | 8/38 (21,0) | 4/38 (10,5) | 2/38 (5,3) + |

| | | | | | |
|---|---|---|---|---|---|
| + : ces deux animaux sont morts quelques heures après la naissance | | | | | |

## Revendications

1. Méthode de traitement du noyau diploïde d'une cellule donneuse somatique préalablement à son transfert dans le cytoplasme d'un ovocyte receveur pour reconstituer *in vitro* un embryon de mammifère non humain, **caractérisée en ce qu'**elle comprend :
a) la protéolyse ménagée des protéines non-histones, et
b) l'induction d'un gonflement isomorphe dudit noyau.

2. Méthode selon la revendication 1, **caractérisée en ce que** la protéolyse ménagée est réalisée par l'action d'une protéase à sérine.

3. Méthode selon la revendication 2, **caractérisée en ce que** ladite protéase est la trypsine ou la chymotrypsine.

4. Méthode selon une quelconque des revendications 1 à 3, **caractérisée en ce que** le gonflement du noyau est induit par un traitement par un polyanion choisi parmi l'héparine, le dextrane sulfate, ou les acides polyaspartiques de poids moléculaire supérieur à 20 000.

5. Méthode selon une quelconque des revendications 1 à 4, **caractérisée en ce que** le noyau traité est contenu dans la cellule donneuse, et **en ce que** ledit traitement comprend la perméabilisation préalable de la membrane cytoplasmique de ladite cellule.

6. Méthode selon la revendication 5, **caractérisée en ce que** la perméabilisation de la membrane cytoplasmique est effectuée à l'aide d'au moins un agent perméabilisant choisi parmi la lysolécithine, la streptolysine, la saponine, la digitonine.

7. Méthode de reconstitution *in vitro* d'un embryon de mammifère non humain, **caractérisée en ce qu'**elle comprend:
- le traitement du noyau diploïde d'une cellule donneuse somatique par une méthode selon une quelconque des revendications 1 à 6 ;
- le transfert du noyau traité dans le cytoplasme d'un ovocyte receveur en métaphase II ;
- l'activation de l'embryon reconstitué.

8. Méthode de reconstitution *in vitro* d'un embryon de mammifère non humain, **caractérisée en ce qu'**elle comprend :
- le traitement du noyau diploïde d'une cellule donneuse somatique par une méthode selon une quelconque des revendications 1 à 6 ;
- le transfert du noyau traité dans le cytoplasme d'un ovocyte receveur en en état d'interphase.

9. Méthode selon une quelconque des revendications 7 ou 8, **caractérisée en ce que** le noyau de la cellule donneuse est traité par une méthode selon une quelconque des revendications 1 à 4, et le transfert dudit noyau dans le cytoplasme receveur est effectué par microinjection.

10. Méthode selon une quelconque des revendications 7 ou 8, **caractérisée en ce que** le noyau de la cellule donneuse est traité par une méthode selon une quelconque des revendications 5 ou 6, et le transfert dudit noyau dans le cytoplasme receveur est effectué par fusion de la cellule donneuse et du cytoplasme receveur.

11. Méthode selon la revendication 10, **caractérisé en ce que** la fusion est effectuée par choc électrique.

12. Méthode selon une quelconque des revendications 1 à 11, **caractérisée en ce que** ledit mammifère non humain est un ongulé.

13. Méthode selon la revendication 11, **caractérisée en ce que** ledit ongulé est choisi parmi les bovins, les ovins, les caprins et les porcins.

## Patentansprüche

1. Verfahren zur Behandlung des diploiden Kerns einer somatischen Spenderzelle vor ihrer Übertragung in das Zytoplasma eines Oozytenempfängers, um in vitro einen nicht-menschlichen Säugerembryo zu rekonstituieren, **dadurch gekennzeichnet dass** es umfasst:
a) schonende Proteolyse der Nicht-Histon-Proteine und
b) Induktion eines isomorphen Anschwellens des Kerns.

2. Verfahren nach Anspruch 1, **dadurch gekennzeichnet, dass** die schonende Proteolyse durch Wirkung einer Serinprotease durchgeführt wird.

3. Verfahren nach Anspruch 2, **dadurch gekennzeichnet, dass** die Protease Trypsin oder Chymotrypsin ist.

4. Verfahren nach einem der Ansprüche 1 bis 3, **dadurch gekennzeichnet, dass** Anschwellen des Kerns durch eine Behandlung durch ein Polyanion, ausgewählt unter Heparin, Dextransulfat und den Polyasparaginsäuren mit Molekulargewichten über 20.000, induziert wird.

5. Verfahren nach einem der Ansprüche 1 bis 4, **dadurch gekennzeichnet, dass** der behandelte Kern in der Spenderzelle enthalten ist und dass die Behandlung die vorherige Permeabilisierung der zytoplasmatischen Membran der genannten Zelle umfasst.

6. Verfahren nach Anspruch 5, **dadurch gekennzeichnet, dass** die Permeabilisierung der zytoplasmatischen Membran mit Hilfe wenigstens eines Permeabilisierungsmittels, ausgewählt unter Lysolecithin, Streptolysin, Saponin, Digitonin, durchgeführt wird.

7. Verfahren zur Rekonstitution eines nicht-menschlichen Säugerembryos in vitro, **dadurch gekennzeichnet, dass** es umfasst:
- Behandlung des diploiden Kerns einer somatischen Spenderzelle durch ein Verfahren nach einem der Ansprüche 1 bis 6;
- Übertragung des behandelten Kerns in das Zytoplasma eines Oozytenempfängers in Metaphase II;
- Aktivierung des rekonstituierten Embryos.

8. Verfahren zur Rekonstitution eines nicht-menschlichen Säugerembryos in vitro, **dadurch gekennzeichnet, dass** es umfasst:
- Behandlung des diploiden Kerns einer somatischen Spenderzelle durch ein Verfahren nach einem der Ansprüche 1 bis 6;
- Übertragung des behandelten Kerns in das Zytoplasma eines Oozytenempfängers im Interphasenzustand.

9. Verfahren nach Anspruch 7 oder 8, **dadurch gekennzeichnet, dass** der Kern der Spenderzelle durch ein Verfahren nach einem der Ansprüche 1 bis 4 behandelt wird und die Übertragung des Kerns in das Empfängerzytoplasma durch Mikroinjektion erfolgt.

10. Verfahren nach Anspruch 7 oder 8, **dadurch gekennzeichnet, dass** der Kern der Spenderzelle durch ein Verfahren nach Anspruch 5 oder 6 behandelt wird und die Übertragung des Kerns in das Empfängerzytoplasma durch Fusion der Spenderzelle und des Empfängerzytoplasmas durchgeführt wird.

11. Verfahren nach Anspruch 10, **dadurch gekennzeichnet, dass** die Fusion durch elektrischen Schock durchgeführt wird.

12. Verfahren nach einem der Ansprüche 1 bis 11, **dadurch gekennzeichnet, dass** der nicht-menschliche Säuger ein Huftier ist.

13. Verfahren nach Anspruch 11, **dadurch gekennzeichnet, dass** das Huftier unter Rindern, Schafen, Ziegen und Schweinen ausgewählt wird.

## Claims

1. Method of treating the diploid nucleus of a somatic donor cell prior to transferring it to the cytoplasm of a receptor oocyte for reconstituting a non-human mammalian embryo in vitro, **characterized in that** it comprises:
a) mild proteolysis of the non-histone proteins, and
b) induction of an isomorphous swelling of said nucleus.

2. Method according to Claim 1, **characterized in that** the mild proteolysis is effected by reaction with a serine protease.

3. Method according to Claim 2, **characterized in that** said protease is trypsin or chymotrypsin.

4. Method according to any one of Claims 1 to 3, **characterized in that** the swelling of the nucleus is induced by treatment with a polyanion selected from heparin, dextran sulfate, or polyaspartic acids with a molecular weight above 20,000.

5. Method according to any one of Claims 1 to 4, **characterized in that** the treated nucleus is contained in the donor cell, and **in that** said treatment comprises prior permeabilization of the cytoplasmic membrane of said cell.

6. Method according to Claim 5, **characterized in that** the permeabilization of the cytoplasmic membrane is effected by means of at least one permeabilizing agent selected from lysolecithin, streptolysin, saponin and digitonin.

7. Method of reconstituting a non-human mammalian embryo *in vitro,* **characterized in that** it comprises:
- treatment of the diploid nucleus of a somatic donor cell by a method according to any one of Claims 1 to 6,
- transfer of the treated nucleus to the cytoplasm of a receptor oocyte in metaphase II, and
- activation of the reconstituted embryo.

8. Method of reconstituting a non-human mammalian embryo in vitro, **characterized in that** it comprises:
- treatment of the diploid nucleus of a somatic donor cell by a method according to any one of Claims 1 to 6, and
- transfer of the treated nucleus to the cytoplasm of a receptor oocyte in the interphase state.

9. Method according to Claim 7 or 8, **characterized in that** the nucleus of the donor cell is treated by a method according to any one of Claims 1 to 4, and the transfer of said nucleus to the receptor cytoplasm is effected by microinjection.

10. Method according to Claim 7 or 8, **characterized in that** the nucleus of the donor cell is treated by a method according to Claim 5 or 6, and the transfer of said nucleus to the receptor cytoplasm is effected by fusion of the donor cell and the receptor cytoplasm.

11. Method according to Claim 10, **characterized in that** the fusion is effected by electric shock.

12. Method according to any one of Claims 1 to 11, **characterized in that** said non-human mammal is a hoofed animal.

13. Method according to Claim 11, **characterized in that** said hoofed animal is selected from cattle, sheep, goats and pigs.
